# EUROPEAN PATENT SPECIFICATION

(11) **EP 2 145 632 B1**
(45) Date of publication and mention of the grant of the patent: **23.04.2014**
(21) Application number: 09165557.1
(22) Date of filing: 15.07.2009
(51) Int. Cl.: A61L 15/30, A61L 15/58

(54) **Patch And Patch Preparation**
Pflaster und Pflasterherstellung
Pansement et préparation d'un pansement

(30) Priority: 17.07.2008 JP 2008186562
(43) Date of publication of application: 20.01.2010
(73) Proprietor: Nitto Denko Corporation, Ibaraki-shi Osaka 567-8680 (JP)
(72) Inventor: Funahashi, Miki, Osaka 567-8680 (JP); Hamada, Atsushi, Osaka 567-8680 (JP); Kasahara, Tsuyoshi, Osaka 567-8680 (JP); Ishikura, Jun, Osaka 567-8680 (JP)
(74) Representative: von Kreisler Selting Werner

(56) References cited:
- EP-A2- 0 922 453
- US-A1- 2003 130 427
- US-A1- 2007 051 376

## Description

### TECHNICAL FIELD OF THE INVENTION

The present invention relates to a patch and a patch preparation. More particularly, the present invention relates to a patch and a patch preparation having a superior skin adhesion force, which leave less adhesive residue on the skin.

### BACKGROUND OF THE INVENTION

Patch is a convenient and effective dosage form for wound protection or administration of a drug to the body. The patch is requested to stably maintain the quality for a long time and have good adhesion property.

As a patch having such adhesion property, for example, patent document 1 and patent document 2 disclose patches having an adhesive layer comprised of a crosslinked rubber component having a functional group. In Example 4 of patent document 1, it is described with regard to such patch that oozing of an adhesive in 3 mm or less was observed in the peripheral area of a test piece. Thus, the patches are presumed to have insufficient cohesion force. Moreover, the patches of these documents may have unsatisfactory stability in patch quality due to the presence of the functional group in the rubber component. As shown above, none of the conventional patches sufficiently meet the above-mentioned requirements.

US-A-2007/0051376 discloses a skin-contacting adhesive composition comprising a hydrophobic polymer, such as polyisobutylene.

US-A-2003/0130427 discloses an adhesive composition comprising a hydrophobic polymer such as crosslinked butyl rubbers or polyisobutylene.

EP-A-0922453 discloses percutaneous absorption type preparations comprising a support and a plaster layer thereon. The plaster layer comprises an adhesive such as an acrylic adhesive or a rubber adhesive such as polyisobutylene.

patent document 1: JP-A-03-127727
non-patent document 2: JP-A-10-151185

### Disclosure of the Invention

### Problems to be Solved by the Invention

The present invention has been made in view of the above-mentioned situation and aims to provide a patch having good adhesion property and capable of stably maintaining the quality for a long time.

### Means of Solving the Problems

Accordingly, the present invention provides the following.
(1) A patch comprising a support and an adhesive layer formed on at least one surface of the support, wherein the adhesive layer comprises an elastomer obtained by crosslinking polybutadiene in the presence of an organic peroxide.
(2) The patch of (1), wherein the above-mentioned adhesive layer further comprises a fatty acid alkylester.
(3) The patch of (1) or (2), wherein the above-mentioned adhesive layer further comprises a tackifier.
(4) A patch preparation comprising the patch of any of (1) to (3) and a drug in the adhesive layer of the patch.

### Effect of the Invention

According to the present invention, a patch superior in the skin adhesion force, which leaves less adhesive residue on the skin and stably maintains the quality for a long time, can be obtained. Accordingly, the patch of the present invention is suitable for adhesion to the skin of mammals. In addition, when the patch of the present invention is used for, for example, a patch preparation, an undesirable reaction between a drug and an adhesive composition can be reduced while maintaining good adhesion property, and the quality of the drug can be stably maintained for a long time.

### Best Mode for Carrying out the Invention

A preferable embodiment of the present invention is shown below. The detailed explanation thereof and particular embodiment are solely intended for exemplification purposes and do not limit the scope of the present invention. The explanation of the preferable embodiment below is for exemplification purposes and does not intend to limit the present invention, application thereof and use thereof.

The patch of the present invention can employ a constitution comprising a support and an adhesive layer formed on at least one surface of the support. While the support is not particularly limited, a support substantially impermeable to the components in the adhesive layer, for example, adhesive, additive, drug and the like, namely, a support that does not permit them to pass through the support and be lost from the back face thereof, is preferable.

Examples of such support include single films of polyester, nylon, Saran (registered trade mark), polyethylene, polypropylene, polyvinyl chloride, ethylene-ethyl acrylate copolymer, polytetrafluoroethylene, Surlyn (registered trade mark), metal foil and the like, a laminate film thereof and the like. While the thickness of the support is not particularly limited, it is generally 10-500 µm, preferably 10-200 µm.

Among those, to improve the adhesion force (anchoring force) between the support and the adhesive layer, the support is preferably a laminate film of non-porous type plastic film recited above and a porous film. In this case, an adhesive layer is preferably formed on the porous film side.

As such porous film, one capable of improving the anchoring force with an adhesive layer is employed. Specifically, paper, woven fabric, non-woven fabric, knitted fabric, mechanically perforated sheet and the like can be mentioned. From the aspects of handling property and the like, paper, woven fabric and non-woven fabric are particularly preferable from among those.

In consideration of improvement of anchoring force, flexibility of the whole patch preparation and adhesion operability and the like, a porous film having a thickness of 10-200 µm is employed. In the case of a thin preparation, such as a plaster type and an adhesive tape type, one having a thickness of 10-100 µm is employed.

When woven fabric or non-woven fabric is used as a porous film, the fabric weight thereof is not particularly limited. It is generally 5-30 g/m², preferably 6-15 g/m².

In the present invention, the most preferable support is a laminate film of a 1.5-6 µm-thick polyester film (preferably, polyethylene terephthalate film) and a polyester (preferably, polyethylene terephthalate) non-woven fabric having a fabric weight of 6-12 g/m².

The adhesive layer contains an elastomer obtained by crosslinking polybutadiene in the presence of an organic peroxide having a structure represented by the formula I:

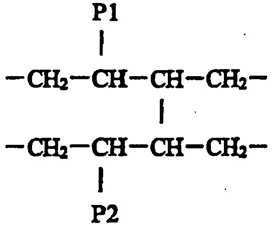

wherein P1 and P2 are each hydrogen.

Here, the above-mentioned elastomer is obtained by, using polybutadiene as a basic polymer, eliminating a carbon-carbon double bond, which is an unsaturated bond, of the basic polymer in the presence of an organic peroxide, and crosslinking the eliminated part between basic polymers by a carbon-carbon single bond. Accordingly, introduction of a functional group into the basic polymer for crosslinking is not necessary. Moreover, the thus-obtained elastomer has a hydrocarbon chain having a comparatively high molecular weight, and can impart skin adhesion force and cohesion force necessary for a patch to the adhesive layer. In addition, the elastomer has a structure wherein hydrocarbon chains are intricately entangled three-dimensionally, and affords an elastomer having a high gel fraction. As a result, a cohesion force sufficient for a patch can be imparted to the adhesive layer.

The basic polymer is polybutadiene, having an unsaturated hydrocarbon chain represented by the formula II:

-CH₂-CH=CH-CH₂-

While the weight-average molecular weight of such polymer is not particularly limited, it is preferably 50,000-5,000,000. The weight-average molecular weight means a value measured by gel permeation chromatography under the following conditions, analysis conditions
GPC apparatus: HLC8120 (manufactured by Tosoh Corporation)
column: TSKgel GMH-H(S) (manufactured by Tosoh Corporation)
standard: polystyrene
eluent: tetrahydrofuran flow rate: 0.5 ml/min
measurement temperature: 40°C
detector: differential refractometer

When polybutadiene is used as a basic polymer, an elastomer having the structure of the formula I as a repeat unit, or an elastomer mainly containing the structure of the formula I, is obtained.

In addition, the above-mentioned elastomer is preferably contained in the adhesive layer in a proportion of 10 wt%-80 wt%, particularly preferably 30 wt%-60 wt%, from the aspects of maintenance of cohesion force and the like.

Organic peroxide is not particularly limited, and those known per se which are generally used in the field of polymerchemical can be used. For example, diacyl peroxide (e.g., dibenzoyl peroxide, diisobutyryl peroxide, di(3,5,5-trimethylhexanoyl) peroxide, dilauroyl peroxide, disuccinic acid peroxide), peroxy esters (e.g., 1,1,3,3-tetramethylbutyl peroxy-2-ethylhexanoate, 2,5-dimethyl-2,5-di(2-ethylhexanoylperoxy)hexane), t-hexyl peroxy-2-ethylhexanoate, t-butyl peroxy-2-ethylhexanoate), ketone peroxide (e.g., methylethylketone peroxide), peroxyketal (e.g., 1,1-di(t-butylperoxy)cyclohexane), hydroperoxide (e.g., p-mentan hydroperoxide), dialkyl peroxide (for example, dicumyl peroxide), peroxydicarbonate (e.g., di-n-propylperoxydicarbonate) and the like can be mentioned.

Of these, from the aspects of reactivity, diacyl peroxide (particularly dibenzoyl peroxide) and peroxy ester (particularly 1,1,3,3-tetramethylbutyl peroxy-2-ethylhexanoate) are preferable. The content of organic peroxide is preferably 0.1-5 parts by weight per 100 parts by weight of the basic polymer. When the content of organic peroxide is less than 0.1 part by weight per 100 parts by weight of the basic polymer, the cohesion force may become insufficient, and when it exceeds 5 parts by weight, the adhesive layer becomes hard and the adhesive force may become insufficient.

As mentioned above, an elastomer obtained by crosslinking polybutadiene in the presence of an organic peroxide having a structure of the formula I of the present invention has a structure wherein a hydrocarbon chain is intrinsically entangled. An adhesive layer containing such an elastomer has a sufficient cohesion force. Therefore, the patch of the present invention can contain a large amount of other components, such as a tackifier and the like in the adhesive layer. As a result, the patch of the present invention can more improve adhesive force and tackiness during adhesion to the skin. On the other hand, when the patch of the present invention is adhered to the skin and later detached, the possibility of leaving the adhesive layer on the skin surface is small.

As the tackifier, those known in the field of patch is appropriately selected and used. Examples of the tackifier include petroleum resin (e.g., aromatic petroleum resin, aliphatic petroleum resin), terpene resin, rosin resin, coumaroneinden resin, styrene resin (e.g., styrene resin, α-methylstyrene), hydrogenated petroleum resin (e.g., alicyclic saturated hydrocarbon resin) and the like. Among these, alicyclic saturated hydrocarbon resin is preferable since the preservation stability of the drug becomes fine.

Tackifiers can be used in a combination of one or more kinds thereof. When two or more kinds are used in combination, for example, resins having different kinds and softening points may be combined.

The content of tackifier is preferably 10-180 parts by weight, more preferably 30-90 parts by weight, per 60 parts by weight of the basic polymer. When the content of tackifier is less than 10 parts by weight, the tackiness and cohesion force may become poor, and when it exceeds 180 parts by weight, the adhesive layer tends to be too soft and sticky.

In addition, in the patch of the present invention, since the adhesive layer has a sufficient cohesion force as mentioned above, the adhesive layer can contain a large amount of organic liquid components. As a result, the patch of the present invention can provide a soft feeling upon adhesion to the skin, and low irritation upon detachment from the skin. In this case, even when the patch of the present invention is released after adhesion to the skin, the possibility of the adhesive layer remaining on the skin surface is small. In addition, when the adhesive layer contains the below-mentioned drug, the transdermal absorption thereof can be promoted.

As the organic liquid component, a hydrophobic liquid component is preferable, such as fatty acid alkylester, from the aspect of compatibility with the adhesive layer.

Examples of the fatty acid alkylester include fatty acid alkylester comprised of higher fatty acid having 12-16 carbon atoms, preferably 12-14 carbon atoms, and lower monovalent alcohol having 1-4 carbon atoms. The above-mentioned higher fatty acid includes lauric acid (C12), myristic acid (C14) or palmitic acid (C16), preferably myristic acid. Examples of the above-mentioned monovalent alcohol include methyl alcohol, ethyl alcohol, propyl alcohol, butyl alcohol and the like, preferably isopropyl alcohol. Accordingly, preferable fatty acid alkyl ester is isopropyl myristate. The organic liquid components may be used in combination of one or more kinds thereof.

The content of organic liquid component is preferably 3-240 parts by weight, more preferably 4-150 parts by weight, per 60 parts by weight of the basic polymer. When the content of organic liquid component is less than 3 parts by weight, a superior soft feeling is imparted to the adhesive layer and, when the adhesive layer contains a drug, a high transdermal proabsorptive effect can be obtained. When it is not more than 240 parts by weight, a superior soft feeling is imparted to the adhesive layer and, when the adhesive layer contains a drug, a high transdermal proabsorptive effect can be advantageously obtained while suppressing a decrease in the adhesive force and cohesion force of the whole adhesive layer.

The adhesive layer may further contain other additives known per se, for example, anti-aging agent, antioxidant, UV absorber, filler and the like, as necessary.

The thickness of the adhesive layer is preferably 10 µm-1000 µm, particularly preferably 20 µm-500 µm, from the aspects of skin adhesiveness.

The present invention also relates to a patch preparation containing a drug in the adhesive layer. As mentioned above the patch of the present invention contains an elastomer obtained by crosslinking polybutadiene in the presence of an organic peroxide having a structure of the formula I in the adhesive layer, and has a structure wherein polybutadiene is crosslinked by a carbon-carbon single bond without using a functional group. As a result, the need to consider an undesired reaction of the functional group and the drug can be reduced, thus increasing the degree of freedom in the design of the patch preparation. In addition, since the number of carbon-carbon double bonds in the basic polymer decreases due to the carbon-carbon single bond between basic polymers, the reaction points decreases, thus possibly contributing to the stability of the drug.

The drug here is not particularly limited, and one permitting administration to mammals such as human and the like through the skin thereof, i.e., a transdermally absorbable drug is preferable. Specific examples of such drug include general anesthetics, hypnotic sedatives, antiepileptic drugs, antipyretic analgesic antiphlogistic drugs, anti-vertiginous drugs, psychoneurotic drugs, topical anesthetics, skeleton muscle relaxants, autonomic drugs, antiepileptic drugs, anti-parkinsonian drugs, anti-histamine drugs, cardiac stimulants, drugs for arrhythmia, diuretics, hypotensive drugs, vasoconstrictors, coronary vasodilators, peripheral vasodilators, arteriosclerosis drugs, drugs for circulatory organ, anapnoics, antitussive expectorants, hormone drugs, external drugs for mattery diseases, analgesic-antipruritic-styptic-antiphlogistic drugs, drugs for parasitic dermatic diseases, drugs for arrest of bleeding, gout treatment drugs, drugs for diabetes, drugs for anti-malignant tumor, antibiotics, chemical therapy drugs, narcotics, quit smoking aids and the like.

While the content of the drug is not particularly limited as long as it satisfies an effect of transdermally absorbable drugs and does not impair the adhesion property of the adhesive, it is, for example, 0.5-50 parts by weight per 60 parts by weight of the basic polymer.

In addition, the patch and patch preparation of the present invention can be produced by any method, for example, the following methods.
[1] A method comprising (i) a step of dissolving or dispersing polybutadiene and, where necessary, a tackifier, an organic liquid component, a drug and the like in a solvent, adding organic peroxide and mixing and stirring the mixture; (ii) a step of applying the obtained adhesive solution or dispersion to at least one surface of a support, and drying to form the adhesive layer on the surface of the support; and (iii) a step of forming a release liner on the adhesive layer (i.e., direct coating).
[2] A method comprising (i) a step of applying the above-mentioned adhesive solution or dispersion to at least one surface of a release liner for protection; (ii) a step of drying to form the adhesive layer on the surface of a release liner; (iii) a step of adhering a support to the adhesive layer (i.e., indirect coating).

To promote crosslinking of the polybutadiene in the adhesive layer, the above-mentioned methods [1] and [2] preferably further include, for example, a maturing step. A maturing step can be performed by, for example, heating and preserving at 50-300°C for 10-100 hr.

### Examples

The present invention is explained in more detail in the following by referring to Examples, which are not to be construed as limitative.

### 1. Starting materials of adhesive layer

basic polymer containing unsaturated hydrocarbon chain: BR (polybutadiene) weight-average molecular weight 458,000 basic polymer free of unsaturated hydrocarbon chain: PIB (polyisobutylene) weight-average molecular weight 425,000
organic peroxide (PO): dibenzoyl peroxide
tackifier: alicyclic saturated hydrocarbon resin (softening point 100.5°C)
organic liquid component: isopropyl myristate

### 2. Production of elastomer in flask

Polybutadiene or polyisobutylene and organic peroxide (dibenzoyl peroxide) were blended in toluene as a solvent, mixed and heated at 120°C for 4 hr to give an elastomer. The tensile strength of the obtained elastomer was measured as follows.

### Measurement method

An elastomer solution was applied onto a release liner such that the thickness after drying was 100 µm, and dried to give an adhesive sheet. The adhesive sheet was cut into a rectangle having width 20 mm, length 40 mm to give a sample. The sample was densely wound from one side in the length direction of the rectangle with the side as the central axis to give a cylindrical or round-stick test piece. The stress of the test piece was measured at chuck distance 20 mm, tensile strength 300 mm/min using a tensile tester (manufactured by SHIMADZU Corporation, AUTOGRAPH AG-IS), and the maximum stress up to rupture was compared.

### 3. Patch

### Examples 1-4, Comparative Examples 1, 2

An adhesive solution having a solid content of 20-40 wt% was prepared using toluene as a solvent and at the mixing ratio shown in Table 2. This was uniformly applied to a polyethylene terephthalate film as a release liner such that the thickness after drying the adhesive layer became 100 µm, and dried at 100°C for 5 min. Using a laminate of a polyethylene terephthalate non-woven fabric and a polyethylene terephthalate film as a support, the surface of the polyethylene terephthalate non-woven fabric and the adhesive layer were faced to each other and adhered to give a laminate. The prepared laminate was aged by heating and storing in the presence of nitrogen to give a patch.

### 4. Test method

The patches of Examples 1-4 and Comparative Examples 1, 2 were measured as follows regarding the following evaluation items.

### (1) Adhesive force

In a room at 23°C, 60%RH, the patch was cut into a test piece having a width 12 mm, length 5 cm, a release liner of the test piece was removed, and a test piece was press-adhered to a phenol resin plate (test plate) by one reciprocation of a 2 kg roller. The plate was stood for 30 min under this environment, and the adhesive force of the test piece was measured by a tensile tester by stretching the test piece at a releasing angle of 180° and a releasing rate of 300 mm/minute. In the destruction mode, cohesive failure was G and interface destruction was K. Here, a patch having an adhesive force of not less than 0.2 N/12 mm and free of cohesive failure is preferable.

### (2) Retaining force

In a room at 23°C, 60%RH, the patch was cut into a test piece having a width 10 mm, length 5 cm, a release liner of the test piece was removed, and a test piece was press-adhered to a phenol resin plate (test plate) by one reciprocation of a 2 kg roller. The adhered area then was set to 200 mm². The plate was stood for 20 min under 40°C environment and, under 40°C environment, the surface of the test plate, to which the test piece was not adhered, was fixed so that the test plate would be perpendicular to the floor, a 300 g load was hung from the test piece and the time up to a fall of the test piece from the test plate was evaluated as retaining force. In the destruction mode, cohesive failure was G, interface destruction was K, and partial cohesive failure was K/G. Here, a patch having a retaining force that does not permit a fall in a short time is preferable.

From the results of Table 2, the patches of Examples 1-4 have good adhesive force and good cohesion force as the adhesive layer of patch. Particularly, the patch of Example 2 was superior in the adhesive force, and had an appropriate cohesion force due to its retaining force and preferable property as a patch. In addition, the patches of Examples 1-4 showed interface destruction between the test plate and the adhesive layer of the patch during the adhesive force measurement, and an adhesive residue leaving an adhesive on the test plate was not observed.

In contrast, Comparative Example 1 and Comparative Example 2 showed cohesive failure during the adhesive force measurement, and an adhesive residue leaving an adhesive on the test plate was observed. Also, the cohesion force was also insufficient, and the test piece fell in a short time during the measurement of retaining force, and cohesive failure occurred during the fall of the test piece. They were considered to be attributable to the absence of addition of organic peroxide in Comparative Example 1, which prevented crosslinking of basic polymers having an unsaturated double bond by a carbon-carbon single bond, resulting in insufficient cohesion force. It is considered that since PIB free of a carbon-carbon double bond was used as the basic polymer in Comparative Example 2, the basic polymers were not crosslinked by a carbon-carbon single bond, or crosslinking was insufficient to result in insufficient cohesion force.

### 5. Patch preparation

### Examples 5-8

In the same manner as in the above-mentioned Examples 1-4, except that 1 part by weight is subtracted from 35-60 parts by weight of each basic polymer in Examples 1-4 and 1 part by weight of indomethacin is added as a drug, the patch preparations of Examples 5-8 are prepared.

The patch preparations of Examples 5-8 have the properties similar to those of the patches of the above-mentioned Examples 1-4.

**Table 1**

| | composition | | | | | | measurement | | | | | |
|---|---|---|---|---|---|---|---|---|---|---|---|---|
| | basic polymer kind | basic polymer: tackifier (weight ratio) | basic polymer (parts by weight) | tackifier (parts by weight) | organic liquid component (parts by weight) | amount (parts by weight) of organic peroxide per 100 parts by weight of basic polymer | adhesive force [N/12 mm] | | | adhesion [min] | | |
| | | | | | IPM | | average | destruction | evaluation | average | destruction | evaluation |
| Ex. 1 | polybutadiene | 1:1 | 35 | 35 | 30 | 0.2 | 1.30 | K | ⊚ | 5.17 | K/G | ⊚ |
| Ex. 2 | polybutadiene | 1:1 | 35 | 35 | 30 | 0.3 | 0.72 | K | ⊚ | 7.40 | K/G | ⊚ |
| Ex. 3 | polybutadiene | 1:1 | 35 | 35 | 30 | 0.35 | 0.64 | K | ⊚ | 10.10 | K/G | ⊚ |
| Ex. 4 | polybutadiene | 1:1 | 35 | 35 | 30 | 0.4 | 0.55 | K | ○ | 24h< | - | ○ |
| Ex. 5 | polybutadiene | 3:1 | 52.5 | 17.5 | 30 | 0.3 | 0.14 | K | Δ | 24h< | - | ○ |
| Ex. 6 | polybutadiene | 3:1 | 52.5 | 17.5 | 30 | 0.4 | 0.16 | K | Δ | 24h< | - | ○ |
| Ex. 7 | polybutadiene | 2:1 | 46.7 | 23.3 | 30 | 0.3 | 1.42 | K | ⊚ | 103.10 | K/G | ⊚ |
| Ex. 8 | polybutadiene | 1:1.25 | 31.1 | 38.9 | 30 | 0.3 | 0.80 | K | ⊚ | 2.70 | K/G | ⊚ |
| Ex. 9 | polybutadiene | 1:1.5 | 28.0 | 42.0 | 30 | 0.3 | 0.83 | K | ⊚ | 2.90 | K/G | ⊚ |
| Ex. 10 | polybutadiene | 3:1 | 60 | 20 | 20 | 0.2 | 1.59 | K | ⊚ | 24h< | - | ○ |
| Comp. Ex. 1 | polybutadiene | 3:1 | 60 | 20 | 20 | 0 | 1.00 | G | × | 9.90 | G | × |
| Comp. Ex. 2 | polyisobutylene | 3:1 | 60 | 20 | 20 | 1 | 1.74 | G | × | 1.27 | G | × |

**Table 2**

| | basic polymer/amount (parts by weight) | organic peroxide (parts by weight) | | tackifier (parts by weight) | organic liquid component (parts by weight) | adhesive force (N/12 mm)/ destruction mode | adhesion (min)/ destruction mode |
|---|---|---|---|---|---|---|---|
| | | amount (parts by weight) | amount (parts by weight) per 100 parts by weight of basic polymer | | | | |
| Ex. 1 | BR/60 | 0.6 | 1 | 20 | 20 | 0.79/K | did not fall for 24 hr or longer |
| Ex. 2 | BR/35 | 0.0875 | 0.25 | 35 | 30 | 0.92/K | 5.2/K/G |
| Ex. 3 | BR/35 | 0.175 | 0.5 | 35 | 30 | 0.59/K | did not fall for 24 hr or longer |
| Ex. 4 | BR/35 | 0.35 | 1 | 35 | 30 | 0.48/K | did not fall for 24 hr or longer |
| Comp. Ex. 1 | BR/60 | 0 | 0 | 20 | 20 | 1/G | 9.9/G |
| Comp. Ex. 2 | PIB/60 | 0.6 | 1 | 20 | 20 | 1.74/G | 1.27/G |

### Industrial Applicability

The patch of the present invention stably maintains the quality for a long time, is superior in the skin adhesion force, is associated with less adhesive residue on the skin surface, and can be utilized for a patch preparation.

## Claims

1. A patch comprising a support and an adhesive layer formed on at least one surface of the support, wherein the adhesive layer comprises an elastomer obtained by crosslinking polybutadiene in the presence of an organic peroxide.

2. The patch of claim 1, wherein the adhesive layer further comprises a fatty acid alkylester.

3. The patch of claim 1 or 2, wherein the adhesive layer further comprises a tackifier.

4. A patch preparation comprising the patch of any of claims 1 to 3 and a drug in the adhesive layer of the patch.

## Patentansprüche

1. Pflaster, umfassend einen Träger und eine auf wenigstens einer Fläche des Trägers gebildete Kleberschicht, wobei die Kleberschicht ein Elastomer umfasst, das durch Vernetzen von Polybutadien in Gegenwart eines organischen Peroxids erhalten wird.

2. Pflaster gemäß Anspruch 1, wobei die Kleberschicht weiterhin einen Fettsäurealkylester umfasst.

3. Pflaster gemäß Anspruch 1 oder 2, wobei die Kleberschicht weiterhin einen Klebrigmacher umfasst.

4. Pflasterpräparat, umfassend das Pflaster gemäß einem der Ansprüche 1 bis 3 und einen Wirkstoff in der Kleberschicht des Pflasters.

## Revendications

1. Patch comprenant un support et une couche adhésive formée sur au moins une surface du support, dans lequel la couche adhésive comprend un élastomère obtenu par réticulation de polybutadiène en présence d'un peroxyde organique.

2. Patch selon la revendication 1, dans lequel la couche adhésive comprend en outre un ester alkylique d'acide gras.

3. Patch selon la revendication 1 ou 2, dans lequel la couche adhésive comprend en outre un agent d'adhésivité.

4. Préparation de patch comprenant le patch selon l'une quelconque des revendications 1 à 3 et un médicament dans la couche adhésive du patch.
